(19) Europäisches Patentamt
European Patent Office
Office européen des brevets

(11) Publication number: 0 336 314 B1

(12) # EUROPEAN PATENT SPECIFICATION

(45) Date of publication of patent specification: 29.09.93

(51) Int. Cl.[5]: C07C 253/08, C07C 255/04

(21) Application number: 89105705.1

(22) Date of filing: 31.03.89

(54) **Promoter synergism in pentenenitrile hydrocyanation.**

(30) Priority: 31.03.88 US 176241

(43) Date of publication of application:
11.10.89 Bulletin 89/41

(45) Publication of the grant of the patent:
29.09.93 Bulletin 93/39

(84) Designated Contracting States:
BE DE FR GB IT NL

(56) References cited:
EP-A- 0 248 643
US-A- 3 496 217
US-A- 3 496 218
US-A- 4 774 353

CHEMICAL ABSTRACTS, vol. 101, no. 11, 10 September 1984, page 568, abstract no. 90011k, Columbus, Ohio, US; W.C. SEIDEL et al.: "Homogeneous nickel-catalyzed olefin hydrocyanation"

CHEMICAL ABSTRACTS, vol. 100, no. 8, February 1984, page 514, abstract no. 50763e, Columbus, Ohio, US; C.A. TOLMAN et al.: "Catalytic hydrocyanation of olefins by nickel(0) phosphite complexes - effects of Lewis acids"

(73) Proprietor: E.I. DU PONT DE NEMOURS AND COMPANY
1007 Market Street
Wilmington Delaware 19898(US)

(72) Inventor: McKinney, Ronald James
1243 Lakewood Drive
Wilmington Delaware 19803(US)
Inventor: Osborne, Robert Baldwin
2209 Summer Oak Lane
Orange Texas 77630(US)

(74) Representative: Abitz, Walter, Dr.-Ing. et al
Abitz, Morf, Gritschneder, Freiherr von Wittgenstein Postfach 86 01 09
D-81628 München (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3. 10/3.6/3. 3. 1)

## Description

This invention concerns an improved process for producing adiponitrile (ADN) by the zerovalent nickel catalyzed hydrocyanation of pentenenitriles (PN), wherein the improvement comprises utilizing a combination of two catalyst promoters specially selected on the basis of the reaction kinetics of the synthesis.

The preparation of dinitriles, such as ADN, from mononitriloolefins, such as PN, utilizing zerovalent nickel catalysts with or without a catalyst promoter is widely practiced in the art. The preparation of ADN from PN, is of particular interest because ADN is an intermediate used in the production of hexamethylenediamine which is in turn used to produce polyhexamethyleneadipamide, a commercial polyamide useful in forming fibers, films and molded articles.

U.S. Pat. 2,571,099, issued on October 16, 1951 to Paul Arthur, Jr. et al. discloses the use of nickel carbonyl with or without the addition of a tertiary aryl phosphine or arsine. This process produces a relatively high percentage of undesirable polymeric products when applied to nonconjugated olefinic starting materials and a relatively poor yield in all cases.

U.S. Pat. 3,496,215, issued on February 17, 1970 to W.C. Drinkard et al., discloses an improvement in nickel-catalyzed hydrocyanation wherein triarylphosphite ligands are utilized and carbonyl ligands are eliminated, thereby dramatically reducing polymer formation and generally increasing yield to desirable nitrile products.

The teaching of the use of a promoter in the hydrocyanation reaction appears in U.S. Pat. 3,496,217 issued on February 17, 1970 to W.C. Drinkard et al. This patent discloses an improvement in hydrocyanation using a promoter selected from a large number of metal cation compounds with a variety of anions as catalyst promoters. More particularly, the patent discloses as a promoter a cation of zinc, cadmium, beryllium, aluminum, gallium, indium, silver, titanium, zirconium, hafnium, germanium, tin, vanadium, niobium, scandium, chromium, molybdenum, tungsten, manganese, rhenium, palladium, thorium, erbium, iron and cobalt, or mixtures thereof. Preferred anions are halide, i.e., fluoride, chloride, bromide, and iodide; anions of lower fatty acids of from 2 to 7 carbon atoms, $HPO_3^{-2}$ , $H_2PO_2^-$ , $CF_3COO^-$, $OSO_2C_7F_{15}^-$ and $SO_4^{-2}$ , etc. The known organometallic compounds $(C_2H_5)_3Al_2Cl_3$, and $C_2H_5A1C1_2$ are also disclosed as promoters.

U.S. Pat. 3,496,218 issued on February 17, 1970 to W.C. Drinkard discloses a nickel hydrocyanation catalyst promoted with various boron-containing compounds, including triphenylboron and alkali metal borohydrides.

U.S. Pat. 3,925,445 issued on December 9, 1975 to C.M. King et al. discloses zerovalent nickel hydrocyanation catalysts promoted with metal halides and organoboron compounds.

U.S. Pat. 3,852,325 issued on December 3, 1974 to C.M. King teaches that along with production of 3-pentenenitrile (3PN) in the hydrocyanation of butadiene there is also obtained varying amounts of cis-and trans-2-pentenenitriles (C-2PN and T-2PN) and that these 2-pentenenitriles are found to be detrimental to catalyst efficiency in the hydrocyanation of 3PN or 4-pentenenitrile (4PN) to adiponitrile (ADN). The patentee also teaches that T-2PN cannot be removed satisfactorily from a mixture of pentenenitriles by fractional distillation, for example, because its boiling point is too close to that of other pentenenitriles such as 3PN or 4PN. Isomerizing T-2PN to the more volatile C-2PN which in turn can be removed from the reaction mixture by fractional distillation is discussed.

Commonly assigned application Serial No. 870,895, filed June 5, 1986, discloses a process for the preparation of dinitriles, e.g., ADN, from unsaturated nitriles, e.g., PN, in the presence of a zerovalent nickel catalyst and a triorganotin catalyst promoter having the general formula

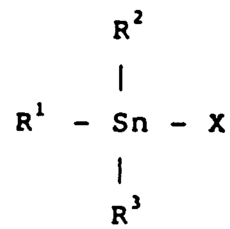

wherein $R^1$, $R^2$ and $R^3$ are the same or different and are selected from groups consisting of alkyl and substituted aryl groups of 6 to 16 carbon atoms. One or more of the substituent groups in the substituted alkyl and aryl groups may be selected from fluoro or alkoxy groups having 1 to 8 carbon atoms, trialkylsilyl

groups with alkyls having 1 to 6 carbon atoms, cyanoalkyl groups having 1 to 20 carbon atoms, and sulfonato. The anion X is a non-nucleophilic ion, the conjugate acid of which has a $pK_a$ less than about 4.

The present invention is an improvement in the process for producing adiponitrile by the addition of hydrogen cyanide to pentenenitriles in the presence of a zerovalent nickel catalyst, and catalyst promoter, the improvement comprising conducting the process in the presence of a combination of two Lewis acid promoters, $LA_A$ and $LA_B$, wherein the rate constant for the rate of isomerization of 3-pentenenitrile to 4-pentenenitrile, $k_1$ in the presence of the first Lewis acid promoter, $LA_A$, is greater than $k_1$ in the presence of the second Lewis acid promoter, $LA_B$, and wherein the rate constant for the rate of hydrocyanation of 4-pentenenitrile to adiponitrile, $k_2$, in the presence of $LA_B$ is greater than $k_2$ in the presence of $LA_A$.

While a wide variety of Lewis acid promoters known in the art and described therein are useful when selected in accordance with this invention, preferable promoter combinations include $LA_A$ being triphenylboron ($BPh_3$) when $LA_B$ selected from $Ph_3SnO_3SCF_3$, $Ph_3SnPh_3BCN$ and $RA1C1_2$ or $LA_A$ being $ZnCl_2$ when $LA_B$ is one of $Ph_3SnO_3SCF_3$ or $RAlCl_2$, where R is an alkyl group of from 1 to 15 carbon atoms.

Use of a combination of promoters in accordance with this invention increases catalyst activity to an extent that reduces the total amount of promoter and/or catalyst fed to the system and minimizes the yield of undesirable 2PN, a well-known cause of yield loss and catalyst inhibition.

Furthermore, because undesirable 2PN yield is reduced, conversion may be dramatically increased and recycle reduced. Because activity is greater, the steady state concentration of HCN may be kept lower, resulting in less catalyst degradation and, therefore, a reduction in the amount of solids in the process streams.

In the zerovalent nickel catalyzed hydrocyanation of PN to produce ADN, the synthesis proceeds as follows:

$$(1)\quad CH_3-CH=CH-CH_2-CN \xrightarrow{k_1} CH_2=CH-CH_2-CH_2-CN$$

3-pentenenitrile (3PN) 4-pentenenitrile (4PN)

Where $k_1$ = rate constant for reaction (1)

$$(2)\quad \underset{4PN}{CH_2=CH-CH_2-CH_2-CN} + HCN \xrightarrow{k_2} \underset{ADN}{NC-CH_2-CH_2-CH_2-CH_2-CN}$$

Where $k_2$ = rate constant for reaction (2)

In accordance with this invention, it was discovered that some promoters, e.g. $BPh_3$, are better for promoting the isomerization of reaction (1), whereas other promoters, e.g. $Ph_3SnO_3SCF_3$, are relatively more active for promoting the hydrocyanation of reaction (2). A selected combination of such promoters leads to a higher steady state concentration of 4PN than the more active hydrocyanation promoter of reaction (2) would normally encounter in single promoter systems or in systems containing incorrectly selected promoter combinations. Thus, combinations of promoters in accordance with this invention provides a synergistic increase in the overall synthesis rate.

The rate of isomerization of 3PN to 4PN, reaction (1), is defined by the rate expression

$$d[4PN]/dt = k_1 \text{ X } [3PN][NiL_4][HCN][LA]/[L]^2$$

whereas the rate of hydrocyanation of 4PN to ADN, reaction (2) is defined by the rate expression

$$d[ADN]/dt = k_2 \text{ X } [4PN][NiL_4][HCN][LA]/[L]^2$$

wherein

LA = Lewis acid promoter

L = neutral ligand

Designating two different Lewis acids as $LA_A$ and $LA_B$, effective combinations of Lewis acids will exhibit rate synergism when:

$k_1\ (LA_A) > k_1\ (LA_B)$ and $k_2\ (LA_B) > k_2\ (LA_A)$.

The rate constants $k_1$ and $k_2$ can be determined for a selected Lewis acid by contacting a solution of zerovalent nickel catalyst in 3PN with a solution of the Lewis acid promoter in 3PN, heating the resulting solution in an inert atmosphere, to a temperature from about 0°C to about 75°C, injecting HCN into the heated solution to commence the hydrocyanation, removing aliquots of the resulting solution at measured time intervals, and analyzing the aliquots for the concentration of the components of the aliquot. The analysis results in a concentration versus time profile of the components of the aliquots, in particular 4PN and ADN.

Kinetic modeling using an interactive GEAR integration computer program (GIT) allowed the extraction of the desired rate constants on the basis of the concentration versus time data. (See Weigert, F.J. Computers in Chemistry, Volume 11, page 273, 1987; GIT is available through Quantum Chemistry Program Exchange, QCPE, Department of Chemistry, Indiana University, Bloomington, Indiana; QCPE #552 (VAX version); QCMP #022 (PC version); ref. QCPE Bulletin Vol. 6, No. 4, pgs 136, 141). The following chemical equations were used in the kinetic modeling procedure:

$$NiL_4 + HCN + LA \rightleftharpoons HNi(CN.LA)L_2 + 2L$$

$$3PN + HNi(CN.LA)L_2 \rightleftharpoons 4PN + HNi(CN.LA)L_2$$

$$3PN + HNi(CN.LA)L_2 \rightleftharpoons 2PN + HNi(CN.LA)L_2$$

$$4PN + HNi(CN.LA)L_2 \longrightarrow ADN + NiL_2 + LA$$

$$4PN + HNi(CN.LA)L_2 \longrightarrow MGN + NiL_2 + LA$$

$$NiL_2 + 2L \longrightarrow NiL_4$$

where $NiL_4$ is the nickel catalyst, $HNi(CN.LA)L_2$ is a possible catalytic intermediate, L is a neutral ligand, 3PN, 4PN and 2PN are 3-, 4- and 2-pentenenitriles, ADN is adiponitrile and MGN is methylglutaronitrile. This model gives satisfactory fits with experimental data. However, improved fits may result with inclusion of catalyst degradation pathways in the model, especially for later data points.

The catalysts employed for hydrocyanation are zerovalent nickel (Ni(0)) compounds free of carbon monoxide which may be preformed or prepared in situ and include nickel compounds containing ligands such as alkyl or aryl groups (either of which groups can contain up to 18 carbon atoms) phosphines, arsines, stibines, phosphites, arsenites, stibites, and mixtures thereof.

An especially preferred group of these Ni(0) compounds have the general structure:

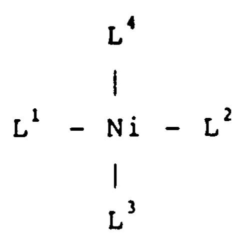

where $L^1$, $L^2$, $L^3$, and $L^4$ are neutral ligands which may be the same or different and have the formula P-(XYZ) wherein X and Y are selected from the class consisting of R and OR, and Z has the formula OR, wherein the three R's may be the same or different, and wherein R is selected from the class consisting of alkyl and aryl groups containing up to 18 carbon atoms with aryl.

A particularly preferred group within the foregoing zerovalent nickel catalysts are those found in U.S. Pat. No. 3,903,120 issued on September 2, 1975 which can be described by the general formula $NiL_4$ where L is a neutral ligand such as a triaryl phosphite of the formula $P(OAr)_3$ wherein Ar is an aryl group of up to 18 carbon atoms. Illustrative of the aryl groups are methoxyphenyl, tolyl, xylyl and phenyl. Meta- and para-tolyl and mixtures thereof are the preferred aryl groups. Excess ligand can be employed.

The promoters useful in the practice of this invention can be selected in combination in accordance with this invention from a wide variety of promoters known in the art. Particularly useful are those described in U.S. Pat. 3,496,217, U.S. Pat. 3,496,218, U.S. Pat. 3,925,445 and commonly assigned application Serial No.

870,895 filed June 5, 1986. Of these promoters, $BPh_3$, $ZnCl_2$, $Ph_3SnO_3SCF_3$, $Ph_3SnPh_3BCN$ and $C_{12}H_{25}AlCl_2$ in combinations selected in accordance with the invention are highly effective. Especially preferred are combinations of $BPh_3$ with one of $Ph_3SnO_3SCF_3$, $Ph_3SnPh_3BCN$ and $C_{12}H_{25}AlCl_2$.

**EXAMPLE A**

Determination of Rate Constants

A nickel catalyst mixture was prepared by dissolving tetrakis(tri-para-tolylphosphite)nickel(0) (0.625 g; 0.426 mmol) and tri-para-tolylphosphite (1.00 mL; 1.15 g; 3.26 mmol) in 3-pentenenitrile (3PN) (49 mL).

For each LA promoter tested (Shown in Table 1), a promoter mixture was prepared by dissolving sufficient promoter in 4.6 mL of 3PN to result in a 0.41 M solution of promoter.

A 4.0 mL aliquot of the nickel catalyst solution was treated with 0.030 mL of the promoter solution, sealed in a 5 mL vial under nitrogen with a septum cap and heated in a thermostated oil bath at 52°C. After temperature equilibration, liquid HCN (0.025 mL) was injected through the septum. Aliquots (0.015 mL) of the reaction mixture were removed by syringe at measured time intervals and quenched by dilution in air saturated acetone (1.5 mL). Capillary gas chromatographic analysis (25 m Carbowax) of these samples resulted in a concentration versus time profile of the products of the hydrocyanation, in particular 4PN and ADN.

These data were input to the iterative GEAR program (GIT) to give the values for $k_1$ and $k_2$ shown for the Lewis acids in Table 1.

TABLE 1

| Lewis Acid (LA) | $k_1$ | $k_2$ (liter/mole-sec) |
|---|---|---|
| $BPh_3$ | 0.010 | 0.036 |
| $ZnC1_2$ | 0.0037 | 0.035 |
| $Ph_3SnO_3SCF_3$ | 0.0022 | 0.15 |
| $Ph_3SnO_3SC_6H_4CH_3$ | 0.0007 | 0.023 |
| $Ph_3SnPh_3BCN$ | 0.0068 | 0.050 |
| $(c\text{-}C_6H_{11})_3SnO_3SCF_3$ | 0.0009 | 0.040 |

From these data, in accordance with this invention, we expect the combinations of $BPh_3/Ph_3SnO_3SCF_3$, $ZnC1_2/Ph_3SnO_3SCF_3$, $BPh_3/Ph_3SnPh_3BCN$, and $BPh_3/(c\text{-}C_6H_{11})_3SnO_3SCF_3$ to be synergistic, whereas $BPh_3/ZnCl_2$, $BPh_3/Ph_3SnO_3SC_6H_4CH_3$ and $ZnCl_2/Ph_3SnPh_3BCN$ will not be synergistic. The Examples which follow confirm and illustrate this.

**EXAMPLES 1-14**

**PRODUCTION OF ADN**

All Examples 1-14 below were carried out as single-stage continuous hydrocyanations in a 25 mL glass, continuous, stirred-tank reactor (CSTR). The ingredients were fed from ISCO 314 pump reservoirs and products collected from the reactor overflow for analysis. Catalyst solutions were made up to 6.5-7.5:1 TTP:Ni(0) from Ni(m,p-TTP)$_4$ and m,p-TTP in 3PN (where m,p-TTP refers to tritolylphosphite with a mixture of meta and para tolyl groups). Hydrogen cyanide (purified by distillation) and promoters were fed as concentrated solutions in dried 3-pentenenitrile.

The reactions were monitored by periodic sampling of the reactor for HCN concentration; lower HCN concentration indicates higher activity. The reactions were run for 2 to 4 days to prove stability under steady state conditions. Product analysis was carried out by gas chromatography. Purified acetone was used to homogenize product mixtures which upon cooling showed phase separation, as in the high conversion examples illustrated in Table 3.

Tables 2 and 3 contain the summarized results for low conversion (50% 3,4PN --> DN, DN=dinitrile) (Examples 1-10), and higher conversion (70-85% 3,4PN --> DN) (Examples 11-14), respectively. Synergism in a promoter combination is indicated by a steady state HCN concentration which is lower than that found for use of either promoter alone. "AF Cycles" refers to the number of moles of 3,4PN per moles of promoter fed to the reactor (sum of both promoters where more than one used); a larger number means

less promoter was fed to the reactor. Yields are defined as follows:

$$\% \text{ ADN Yield} = \frac{\text{Moles of ADN produced}}{\text{Moles of 3,4PN reacted}} \times 10^2$$

$$\% \text{ 2PN Yield} = \frac{\text{Moles of 2PN produced}}{\text{Moles of 3,4PN reacted}} \times 10^2$$

**EXAMPLE 1**

A 25 mL multineck glass reactor charged with a $Ni(TTP)_4$/TTP catalyst mixture (3.62 g; about 6.6:1 TTP/Ni(0), $B(C_6H_5)_3$ (0.21 g), 3,4-pentenenitrile (PN's) (9.25 g), and adiponitrile (9.44 g) was warmed to and maintained at 50 °C with a thermostatically controlled heated air gun. The following solutions (wt%) were fed from reservoir pumps in a continuous fashion for 48.6 hours:

| Solution | Feed Rate |
|---|---|
| Catalyst/TTP/PN's; 1.17% Ni(0), 6.6:1 TTP:Ni(0) | 1.577 g/hr |
| HCN/PN's; 18.5% HCN | 2.318 g/hr |
| $BPh_3$/PN's; 20.0% $BPh_3$ | 0.192 g/hr |

Product receivers were changed periodically to facilitate sampling. The productivity of the reaction was 0.55 lb of adiponitrile/gallon-hour HCN concentration was monitored periodically by measuring the cyanide absorption at 2095 $cm^{-1}$ by calibrated infrared spectroscopy in a $CaF_2$ cell. The reaction achieved a steady state level of HCN as indicated in Table 2 along with the corresponding product yields.

**EXAMPLES 2-10**

These were carried out in the manner of Example 1 except that where two promoters were used, an additional solution was prepared and fed simultaneously.

**EXAMPLE 11**

A 25 mL multineck glass reactor charged with $Ni(TTP)_4$/TTP catalyst mixture (5.53 g; about 6.6:1 TTP:Ni(0)), $Ph_3SnOTf$ ($OTf = CF_3SO_3$) (0.72 g, 98.4% purity), 3,4-pentenenitrile (PN's) (2.50 g) and adiponitrile (14.50 g) was warmed to and maintained at 60 °C with a thermostatically controlled heated air gun. The following solutions (wt%) were fed from ISCO reservoir pumps in a continuous fashion for 51.7 hours (6.8 reactor turnovers) with product overflowing into an ambient temperature receiver bottle:

| Solution | Feed Rate |
|---|---|
| Catalyst/TTP/PN's; 1.87% Ni(0), 78.68% TTP (7.0:1 TTP:Ni(0)), 14.68% PN's | 0.848 g/hr |
| HCN/PN's; 32.0% HCN | 1.577 g/hr |
| $Ph_3SnOTf$/PN's; 21.98% $Ph_3SnOTf$ | 0.434 g/hr |
| 3,4-PN's | 0.296 g/hr |

Product receivers were changed periodically to facilitate sampling. The reactor solution HCN concentration was monitored as in Example 1. On cooling, the overflow product separated into two phases; this mixture was homogenized by addition of a small amount of purified acetone and analyzed by gc, lc, and emission spectroscopy to give the results shown in Table 3.

## EXAMPLES 12-14

These were carried out in the manner described in Example 12, except where two promoters were used an additional solution was prepared and fed simultaneously.

**TABLE 2**

**LOW CONVERSION HYDROCYANATIONS***

| Example # or Comp.Exp. #/ Promoter(s) | Mole Ratio of Promoters | AF Cycles | HCN Concentration ppm | %ADN Yield | %2PN Yield |
|---|---|---|---|---|---|
| 1a/$BPh_3$ | ----- | 100 | 290 | 91.7 | 4.5 |
| 1b/$BPh_3$ | ----- | 200 | >5000 | -Rxn Failed- | |
| 2/$Ph_3SnOTf$ | ----- | 197 | 1274 | 83.8 | 1.2 |
| 3a/$BPh_3$ + $Ph_3SnOTf$ | 1.1:1 | 191 | 185 | 87.3 | 1.5 |
| 3b/ " " | 3.1:1 | 188 | 247 | 89.5 | 2.4 |
| 3c/ " " | 3.3:1 | 268 | 553 | 89.3 | 2.3 |
| 4/$Ph_3SnPh_3BCN$ | ----- | 210 | 1373 | 84.9 | 1.2 |
| 5a/$BPh_3$ + $Ph_3SnPh_3BCN$ | 2.8:1 | 201 | 272 | 89.9 | 2.6 |
| 5b/ " " | 5.0:1 | 191 | 339 | 90.8 | 3.6 |
| 6/$Cy_3SnOTf$ | ----- | 194 | 445 | 90.1 | 1.9 |
| 7/$BPh_3$ + $Cy_3SnOTf$ | 10:1 | 190 | 267 | 90.3 | 2.5 |
| 8/$ZnCl_2$ | ----- | 207 | 264 | 81.7 | 1.8 |
| 9/$BPh_3$ + $ZnCl_2$ | 0.9:1 | 191 | 379 | 82.0 | 2.3 |
| 10/$ZnCl_2$ + $Ph_3SnOTf$ | 1.0:1 | 199 | 5 | 78.0 | 1.2 |

OTf = $O_3SCF_3$; Cy=$C_6H_{11}$; Ph=$C_6H_5$.

* Reactions at 0.55 lb ADN/gal.-hr., 50°C, 50 AF cycles Ni(O), 50% 3,4PN --> DN conversion

TABLE 3

| High Conversion Hydrocyanations[a] | | | | | |
|---|---|---|---|---|---|
| Example #/Promoter(s) | Mole Ratio of promoters | AF Cycles Promoters | HCN Concentration ppm | %ADN Yield | %2PN Yield |
| 11/$Ph_3SnO_3SCF_3$ | --- | 97 | 771 | 82.5 | 0.5 |
| 12a/$Ph_3B$ + $Ph_3SnO_3SCF_3$ | 2.6:1 | 98 | 274 | 83.8 | 1.1 |
| 12b/$Ph_3B$ + $Ph_3SnO_3SCF_3$ | 9.5:1 | 102 | 1017 | 87.8 | 1.8 |
| 12c/$Ph_3B$ + $Ph_3SnO_3SCF_3$ | 3.1:1 (50 °C) | 98 | 1332 | 85.6 | 1.0 |
| 13a/$Ph_3B$ + $Ph_3SnPh_3BCN$ | 7.5:1 | 98 | 565 | 88.3 | 2.2 |
| 13b/$Ph_3B$ + $Ph_3SnPh_3BCN$ | 19.1:1 (70% conv.) | 99 | 286 | 90.3 | 4.5 |
| 14a/$Ph_3B$ + $Cy_3SnO_3SCF_3$ | 2.6:1 | 97 | 830 | 91.0 | 2.0 |
| 14b/$Ph_3B$ + $Cy_3SnO_3SCF_3$ | 5.3:1 (72% conv.,2/3 rate) | 97 | 158 | 90.1 | 3.6 |

[a] Reactions at 0.55 lb ADN/gal-hr, 60 °C, 67 AF cycles Ni(O). 85% 3,4 PN --> DN conversion unless otherwise noted

## Claims

1. In a process for producing adiponitrile by the addition of hydrogen cyanide to pentenenitriles in the presence of a zerovalent nickel catalyst and catalyst promoter, the improvement comprising conducting the process in the presence of a combination of two Lewis acid promoters, $LA_A$ and $LA_B$, wherein the rate constant for the rate of isomerization of 3-pentenenitrile to 4-pentenenitrile, $k_1$, in the presence of the first Lewis acid promoter, $LA_A$ is greater than $k_1$ in the presence of the second Lewis acid promoter, $LA_B$, and wherein the rate constant for the rate of hydrocyanation of 4-pentenenitrile to adiponitrile, $k_2$ , in the presence of $LA_B$ is greater than $k_2$ in the presence of $LA_A$.

2. The process of Claim 1 wherein $LA_A$ is $BPh_3$ and $LA_B$ is one selected from $Ph_3SnO_3SCF_3$, $Ph_3SnPh_3BCN$ and $RA1C1_2$, where R is an alkyl group of from 1 to 15 carbon atoms.

3. The process of Claim 1 wherein $LA_A$ is $ZnC1_2$ and $LA_B$ is one selected from $Ph_3SnO_3SCF_3$ and $RA1C1_2$, where R is an alkyl group of from 1 to 15 carbon atoms.

4. The process of Claim 1 wherein $LA_A$ is $BPh_3$ and $LA_B$ is $Ph_3SnPh_3BCN$.

## Patentansprüche

1. Bei einem Verfahren zur Herstellung von Adiponitril durch Zugabe von Cyanwasserstoff zu Pentennitrilen in Gegenwart eines nullwertigem Nickel-Katalysators und eines Katalysator-Beschleunigers umfaßt die Verbesserung die Durchführung des Verfahrens in Gegenwart einer Kombination aus zwei Lewis-Säure-Beschleunigern, $L_A$ und $L_B$, bei der die Geschwindigkeitskonstante für die Geschwindigkeit der Isomerisierung von 3-Pentennitril zu 4-Pentennitril, $k_1$, in Gegenwart des ersten Lewis-Säure-Beschleunigers, $LA_A$, größer ist als k1 in Gegenwart des zweiten Lewis-Säure-Beschleunigers, $LA_B$,und bei der die Geschwindigkeitskonstante für die Geschwindigkeit der Hydrocyanierung von 4-Pentennitril zu Adiponitril, $k_2$, in Gegenwart von $LA_B$ größer ist als $k_2$ in Gegenwart von $LA_A$.

2. Verfahren nach Anspruch 1, bei dem $LA_A$ $BPh_3$ darstellt und $LA_B$ ein Beschleuniger ist, der ausgewählt wird aus $Ph_3Sno_3SCF_3$, $Ph_3SnPh_3BCN$ und $RA1Cl_2$, worin R eine Alkylgruppe mit 1 bis 15 Kohlenstoffatomen darstellt.

3. Verfahren nach Anspruch 1, bei dem $LA_A$ $ZnCl_2$ darstellt und $LA_B$ ein Beschleuniger ist, der ausgewählt wird aus $Ph_3SnO_3SCF_3$ und $RA1Cl_2$, worin R eine Alkylgruppe mit 1 bis 15 Kohlenstoffatomen darstellt.

4. Verfahren nach Anspruch 1, bei dem $LA_A$ $BPh_3$ darstellt und $LA_B$ $Ph_3SnPh_3BCN$ bedeutet.

## Revendications

1. Dans un procédé pour la préparation de l'adiponitrile par l'addition d'acide cyanhydrique à des pentènenitriles en présence d'un catalyseur au nickel de valence nulle et d'activateur de catalyseur, le perfectionnement comprenant la conduite du procédé en présence d'une combinaison de deux acides de Lewis activateurs $LA_A$ et $LA_B$, la constante cinétique $k_1$ régissant la vitesse d'isomérisation du 3-pentènenitrile en 4-pentènenitrile en présence du premier acide de Lewis activateur $LA_A$ étant supérieure à $k_1$ en présence du deuxième acide de Lewis activateur $LA_B$, et la constante cinétique $k_2$ régissant la vitesse de transformation du 4-pentènenitrile en adiponitrile par fixation d'acide cyanhydrique en présence de $LA_B$ étant supérieure à $k_2$ en présence de $LA_A$.

2. Le procédé de la revendication 1 dans lequel $LA_A$ est $BPh_3$ et $LA_B$ est choisi parmi $Ph_3SnO_3SCF_3$, $Ph_3SnPh_3BCN$ et $RAlCl_2$, où R est un groupe alkyle comptant 1 à 15 atomes de carbone.

3. Le procédé de la revendication 1 dans lequel $LA_A$ est $ZnCl_2$ et $LA_B$ est choisi parmi $Ph_3SnO_3SCF_3$ et $RAlCl_2$, où R est un groupe alkyle comptant 1 à 15 atomes de carbone.

4. Le procédé de la revendication 1 dans lequel $LA_A$ est $BPh_3$ et $LA_B$ est $Ph_3Snph_3BCN$.